(19) Europäisches Patentamt
European Patent Office
Office européen des brevets

(11) EP 1 868 633 B1

(12) EUROPEAN PATENT SPECIFICATION

(45) Date of publication and mention of the grant of the patent:
**22.01.2014 Bulletin 2014/04**

(21) Application number: **06740105.9**

(22) Date of filing: **31.03.2006**

(51) Int Cl.:
***C40B 30/02*** *(2006.01)* ***A61K 31/164*** *(2006.01)*
***A61K 31/00*** *(2006.01)* ***C07K 1/00*** *(2006.01)*
***A61P 35/00*** *(2006.01)* ***A61P 19/10*** *(2006.01)*
***A61P 19/00*** *(2006.01)* ***A61P 43/00*** *(2006.01)*

(86) International application number:
**PCT/US2006/011754**

(87) International publication number:
**WO 2006/107719 (12.10.2006 Gazette 2006/41)**

(54) **COMPOSITIONS AND METHODS FOR THE INHIBITION OF DISHEVELLED PROTEINS**

ZUSAMMENSETZUNGEN UND VERFAHREN ZUR HEMMUNG VON DISHEVELLED-PROTEINEN

COMPOSITIONS ET PROCEDES PERMETTANT D'INHIBER LES PROTEINES DISHEVELLED

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.04.2005 US 97518**

(43) Date of publication of application:
**26.12.2007 Bulletin 2007/52**

(73) Proprietor: **ENZO BIOCHEM, INC.**
**New York, NY 10022 (US)**

(72) Inventors:
- **ZHENG, Jie**
  **Memphis, TN 38103 (US)**
- **SHAN, Jufang**
  **Forest Hills, NY 11375 (US)**
- **WU, Dianqing**
  **Cheshire, CT 06410 (US)**

(74) Representative: **Vossius & Partner**
**P.O. Box 86 07 67**
**81634 München (DE)**

(56) References cited:
**WO-A2-2004/092346 US-A1- 2003 138 848**

- **KARIM ET AL.: 'The significance of the Wnt pathway in the pathology of human cancers.' PATHOLOGY. vol. 36, no. 2, April 2004, pages 120 - 128, XP008126189**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 868 633 B1

**Description**

REFERENCE TO RELATED PATENT APPLICATIONS

This patent application is a continuation-in-part of the patent application entitled "Sclerostin and the Inhibition on Wnt Signaling and Bone Formation," filed on March 18, 2005 (Dan Wu, et al.). This application is related to the patent application entitled "Compositions and Methods for the Stimulation or Enhancement of Bone Formation and the Self-Renewal of Cells," Application No. 10/849,643, filed on May 19, 2004. This application is also related to the patent application entitled "Compositions and Methods for Bone Formation and Remodeling," Application No. 10/849/067, filed on May 19, 2004.

FIELD OF THE INVENTION

The present invention relates to the Dishevelled proteins, which translate Wnt signals from the transmembrane receptor Frizzled to downstream components in canonical and non-canonical Wnt signaling pathways. The invention relates to the field of therapeutic methods, compositions and uses thereof, in the treatment of various diseases which are caused by Wnt signaling involved in pathogenesis. More particularly, the compositions and methods are directed to compounds that interrupt the Fizzled-Dishevelled interaction. The compounds were identified from libraries of compounds using screening methods. These compounds may also be modified to create derivatives or analogues not found in the libraries or in nature, which also function effectively.

BACKGROUND OF THE INVENTION

Wnt signaling pathways play important roles in embryonic and postembryonic development and have been implicated in tumorigenesis. In the canonical Wnt-β-catenin pathway, secreted Wnt glycoproteins bind to seven-transmembrane domain Frizzled (Fz) receptors and activate intracellular Dishevelled (Dvl) proteins. Activated Dvl proteins then inhibit glycogen synthase kinase-3β (GSK-3β); this inhibition causes destabilization of a molecular complex formed by GSK-3β, adenomatous polyposis coll (APC), axin, and β-catenin and reduces the capability of GSK-3β to phosphorylate β-catenin. Unphosphorylated β-catenin proteins escape from ubiquination and degradation and accumulate in the cytoplasm. This accumulation leads to the translocation of β-catenin into the nucleus, where it stimulates transcription of Wnt target genes, such as the gene encoding the T cell factor/lymphoid enhancer factor (Tcf/Lef). Numerous reports address mutations of Wnt-β-catenin signaling pathway components that are involved in the development of neoplasia.

The link between the Wnt pathway and cancer dates back to the initial discovery of Wnt signaling: the first vertebrate Wnt growth factor was identified as the product of a cellular oncogene (Wnt-1), which is activated by proviral insertion in murine mammary carcinomas. Perhaps the most compelling evidence supporting the role of Wnt signaling in oncogenesis is the finding that approximately 85% of colorectal cancers are characterized by mutations in APC, one of the key components of the Wnt pathway. Members of the Wnt signaling pathway also have been implicated in the pathogenesis of various pediatric cancers such as Burkitt lymphoma, 4 medulloblastoma, Wilms' tumor, and neuroblastoma. Furthermore, aberrant Wnt signaling is involved in other diseases, such as osteoporosis and diabetes.

Dvl relays the Wnt signals from membrane-bound receptors to downstream components and thereby plays an essential role in the Wnt signaling pathway. Dvl proteins are highly conserved throughout the animal kingdom. Three Dvl homologs, Dvl-1, -2, and -3, have been identified in mammalian systems. All three human Dvl genes are widely expressed in fetal and adult tissues including brain, lung, kidney, skeletal muscle, and heart. The Dvl proteins are composed of an N-terminal DIX domain, a central PDZ motif, and a C-terminal DEP domain. Of these three, the PDZ domain appears to play an important role in both the canonical and non-canonical Wnt pathways. Indeed, the PDZ domain of Dvl may be involved not only in distinguishing roles between the two pathways but also in nuclear localization. Recently, the interactions between the PDZ domain (residues 247 through 341) of mouse Dvl-1 (mDvl1) and its binding partners were investigated by using nuclear magnetic resonance (NMR) spectroscopy. The peptide-interacting site of the mDvl1 PDZ domain interacts with various molecules whose sequences have no obvious homology. Although it is not a typical PDZ-binding motif, one peptide that binds to the mDvl1 PDZ domain is the conserved motif (KTXXXW) of Fz, which begins two amino acids after the seventh transmembrane domain. This finding showed that there is a direct interaction between Fz and Dvl and revealed a previously unknown connection between the membrane-bound receptor and downstream components of the Wnt signaling pathways. Therefore, an inhibitor of the Dvl PDZ domain is likely to effectively block the Wnt signaling pathway at the Dvl level.

The special role of the Dvl PDZ domain in the Wnt-β-catenin pathway makes it an ideal pharmaceutical target. Small organic inhibitors of the PDZ domain in Dvl might be useful in dissecting molecular mechanisms and formulating pharmaceutical agents that target tumors or other diseases in which the Wnt signaling is involved in pathogenesis. In light of the structure of the Dvl PDZ domain, virtual ligand screening was used to identify a non-peptide compound, NCI668036, that binds to the Dvl PDZ domain. Further NMR experiments validated that the compound binds to the peptide-binding site on the surface of the PDZ domain; the binding affinity (dissociation constant, $K_D$) of the compound was measured by fluorescence spectroscopy. In addiction, we carried out molecular dynamics (MD) simulations of the interaction between this compound and the PDZ domain as well as that between the C-terminal region of a know PDZ domain inhibitor (Dapper) and the PDZ domain, and we compared the binding free energies of these interactions, which were calculated via the molecular mechanics Poisson-Boltzman surface area (MM-PBSA) method.

WO 2004/092346 discloses that indole derivatives such as sodium [3-hydroxymethyl-2-(1-pentyl)-1-(2-phenylethyl)-5-methyl]indole-6-carboxylate are PD2 domain interaction inhibitors and therefore may be used to treat various cancers.

## SUMMARY OF THE INVENTION

The present invention is based on the activation or inactivation of the intracellular Dishevelled (Dvl) proteins, or homologs of said proteins, which are involved in Wnt signaling pathways.

The present invention provides a compound selected from the group consisting of NCI668036, NCI221120, NCI107146, NCI145882, NCI161613, 8004-1312, 3289-8625, 3289-5066, 3237-0719, 3237-0565, 3237-0713, 3237-0430, 8006-2560, 0090-0031, 2372-2393, 103673, 145882, 3289-5066, 3289-8625, 337837, 7129, 3237-0719, 125217, p1, 142277, 82569, 39869, p3, 46893, 661075, 661080, 661086, 661092, 661091, 84123 or 668036 as shown in Figures 8-11 for use in the treatment of a disease in a mammalian subject, wherein the disease is colorectal cancer, desmoid cancer, endometrial cancer, gastric cancer, hepatocellular cancer, ovarian cancer, pancreatic cancer, prostate cancer, thyroid cancer, uterine cancer, breast cancer, Burkitt's lymphoma, medulloblastoma, Wilms' tumor, neuroblastoma, hepatoblastoma, diabetes, hair loss, bone fracture, bone disease, bone injury, loss of bone mass, a disease of sweat glands, or a disease of mammary glands.

The present invention provides compounds which bind to the Dishevelled proteins or homologs of said Dishevelled proteins to interrupt the interaction of these proteins with Frizzled receptors, or homologs of Frizzled receptors.

The invention provides compounds which bind to the PDZ domain of the Dishevelled proteins to interrupt interactions with transmembrane receptors, such as the Frizzled receptor.

Other aspects of the present invention will be apparent to one of ordinary skill in the art from the following detailed description relating to the present invention.

## DETAILED DESCRIPTION OF THE INVENTION

### Structure-Based Ligand Screening

A search was conducted for potential inhibitors of the PDZ domain of Dvl by the use of structure-based virtual screening. PDZ is a modular protein-interaction domain that has two a helices and six β sheets. The αB helix and βB sheet, together with the loop that proceeds, followed by βB, form a peptide-binding cleft. In their crystal-complex structure, the Dapper peptide (derived from one of the binding partners of the Dvl PDZ domain) forms hydrogen bonds with residues Leu265, Gly266, Ile267, and Ile269 in the βB sheet of the PDZ domain.

To identify small organic compounds that can bind to this groove and interrupt interactions between the PDZ domain and its binding partners, a query was designed by using the program UNITY™, a module in the software package SYBYL™ (Tripos, Inc.). The query consisted of two hydrogen-bond donors (backbone amide nitrogens of Gly266 and Ile269) and two hydrogen-bond acceptors (carbonyl oxygens of Ile267 and Ile269) on the PDZ domain, with 0.3-Å tolerances for spatial constraints. The Flex™ search module of UNITY™ was then used to explore the three-dimensional (3D) small-molecule database of the National Cancer Institute (NCI) to identify compounds that met the requirements of the query. The 3D database is available from NCI at no cost, and it includes the coordinates of more than 250,000 drug-like chemical compounds. The Flex™ search option of UNITY™ considers the flexibility of compounds, and it uses the Directed Tweak algorithm to conduct a rapid and conformationlly flexible 3D search. The search yielded 108 organic compounds as the initial hits.

These 108 hits then were "docked" into the binding site of the PDZ domain using the FlexX™ program of SYBYL™. FlexX™ is energy minimization-modeling software that varies the conformation of the ligand to fit it into the protein-binding site. As a control, we also docked the Dapper peptide into the PDZ domain. The receptor's binding site was defined by residues Gly266, Ile269, and Arg325 with a selection radius of 5.9 Å, and a core sub-pocket was defined by Gly266 with a selection radius of 5.9 Å. Under this condition, the docked Dapper peptide had a similar conformation to that found in crystal structure of the complex with a backbone root mean square deviation (RMSD) of 2.04 Å. In particular, the backbone RMSD for the six C-terminal, amino acids is 1.22 Å, indicating that the docking procedure was able to dock ligand spectroscopy experiments by using fluorophore-labeled PDZ domain (TMR-PDZ). We followed the quenching of fluorescence emission of TMR-PDZ at 579 nm (with the excitation at 552 nm) as we titrated NCI668036 into the TMR-PDZ solution. The fluorescence emission of TMR was quenched because of the binding of NCI668036 to the PDZ domain. A double reciprocal plot of the fluorescence changes against the concentrations of NCI668036 gave a linear correlation. Linear fitting using Origin (Microcal Software, Inc.) calculated a KD (mean $\pm$ standard deviation) of 237 $\pm$ 31 $\mu$M (Fig. 3).

**Molecular Dynamics Simulations of the Complex Between the Dvl PDZ Domain and NCI668036**

To further investigate the interaction between the PDZ domain and NCI668036, the AMBER™ software suit was used to conduct a molecular dynamics (MD) simulation study of the NCI668036-PDZ domain complex. MD simulations were performed in explicit water for 5 ns after equilibration with the particle mesh Ewald (PME) method. The MM-PBSA algorithm was then used to calculate the binding free energy of the interaction between the PDZ domain and NCI668036.

To sample sufficient possible binding modes during the MD simulation, we re-examined the entire output of the initial FlexX™ docking results were re-examined. The default settings of the FlexX™ docking algorithm yielded 30 possible docking conformations (Fig. 4), and the conformed which had the best docking scores were selected. Although the conformations of the 30 docked NCI668036 were very similar overall, there were distinct variations. These 30 bound conformers can be clustered into three main groups. Group I comprises 5 conformers (in red), and the RMSDs of all the atoms in NCI668036 are between 0.46 and 0.77 Å for this group of conformers; group II has 13 conformers (in yellow) with RMSDs between 1.44 and 1.7 Å; and group III has 12 conformers (in blue) with RNLSD between 2.31 to 2.86 Å (Fig. 3A). Manual inspection of these docking conformers led to the selection of 10 conformers as starting points for the MD simulations (see Table 1 for the list of the parameters used in the MD simulations). Of these 10 conformers, one was from group I (conformer 6), five were from group II (conformers 4, 7, 10, 14, and 15), and four were from group III (conformers 12, 22, 26, and 27). During the 10 MD simulation runs, the simulation that started with conformer 22 (group III) had the lowest and most stable binding free energy, suggesting that this conformer represents the true PDZ domain-bound conformation of NCI668036 in solution.

**Structure of the NCI668036-Bound Dvl PDZ Domain**

The MD simulation that started with conformer 22 was analyzed in detail. During the 5-ns MID production run, the total energy of the MD system (waterbox included) fluctuated between -44552.6 kcal $mol^{-1}$ and -44344.2 kcal $mol^{-1}$ (mean, -44450.8 kcal $mol^{-1}$) with a root mean square (rms) of 32.6 kcal mol-$\tau$ (Fig. 5A and 5C). The lowest energy occurs at 4.905 ns; the structure of mDvlI bound with NCI668036 at this point is shown in Fig. 6A. In the complex, NCI668036 formed hydrogen bonds with residues Leu258, Gly259, Ile260, Ile262, and Arg318 of the Dvl PDZ domain (Fig. 6B); close hydrophobic contacts between the ligand and the residues in the PDZ domain were also observed. For example, the valyl group that is connected to carbon C1 was within 3.5 Å of the hydrophobic side chains of residues Leu258, Ile260, Ile262, Leu317, and Val314 as well as the $C_{á}$ side chain of Arg318. In addition, the C17 methyl group was within 3.5 Å of Phe257, and the "C"-terminal t-butyl group had hydrophobic contacts with Val263 and Val314 (within 3.5 A of the hydrophobic side chains of the two residues).

**Bound NCI668036 Adopts a Conformation Similar to That of Bound Dapper Peptide**

A comparison between the crystal structure of the PDZ domain bound with the Dapper peptide and the simulated NCI668036-PDZ domain complex revealed that both ligands adopt similar conformations when bound to the PDZ domain (Fig. 5C and 5D). The mass-weighted backbone RMSD (only the 4 C-terminal amino acids, MTTV, were included in the RMSD calculation) for both the PDZ domain-NCI668036 and the PDZ domain-Dapper peptide was 1.49 Å. The backbone of NCI668036 was defined as the atoms in the main chain between and including the carbonyl carbon of the carboxylate group (C) and the carbonyl carbon at the other end of NCI668036 (C8), (a total of 13 atoms; Fig. 1).

To conduct a further detailed comparison, similar to the MID simulation conducted with the PDZ domain-NCI668036 complex, we first carried out a 5-ns MD simulation for the complex was first carried out which consisted of the PDZ domain and Dapper peptide. For each MD simulation, 1000 "snapshots" were saved and analyzed in detail (Fig. 5). The MD simulations allowed the comparison the hydrogen bonds within the two complexes in depth, and those hydrogen bonds, together with their percentage occupancies in the 1000 snapshots, are listed in Table 5. The most striking difference between the two complexes was within the hydrogen-bond network between the "carboxylate binding loop" formed by the conserved motif of Gly-Leu-Gly-Phe (Phe257-Leu258-Gly259-Ile260 in the mDvl1 PDZ domain) and the C-terminal residue of the bound peptide. This hydrogen-bond network is the hallmark of the structure of a C-terminal peptide complex of a PDZ domain; and in the structure of the Dapper-PDZ domain complex, the amide groups of Leu258, Gly259, and Ile260 donated hydrogen bonds to the carboxylate group of the Dapper peptide. In the NCI668036-PDZ domain complex, because of the flexibility of the ether bond, the C-terminal carboxylate group and oxygen 03 were in cis conformation. This conformation allowed both oxygen 03 and the C-terminal carboxylate group to be involved in the "hydrogen network"; the amide groups of Gly259 and Ile260 form hydrogen bonds with oxygen O3, and the C-terminal carboxylate group of NCI668036 forms a hydrogen bond with the amide group of Leu258. Outside the "carboxylate binding network", the two bound ligands had very similar hydrogen bonds and hydrophobic contacts with the host PDZ domain. Therefore, the increased binding affinity of the Dapper peptide likely is due to the extra length of the peptide-residues Lys5, Leu6, and Ser7 of the bound Dapper peptide form multiple hydrogen bonds and hydrophobic contacts with the host PDZ domain.

To further compare the binding events of the Dapper peptide and NCI668036 to the PDZ domain, the binding free energies of the complexes were examined. The absolute binding free energies for both systems were calculated by using the MM-PBSA approach in combination with the normal mode analysis. The binding free energy was -1.88 kcal $mol^{-1}$ for the PDZ-NCI668036 complex and -7.48 kcal $mol^{-1}$ for the PDZ-Dapper peptide complex (see Tables 2, 3, and 4 for all the energy elements obtained from the MM-PBSA free binding energy . calculations). The relative ranking of binding free energies was consistent with experimental data. Indeed, as the dissociation constants for NCI668036 and the Dapper peptide were 237 $\mu$M and 10 $\mu$M, respectively, at 25 °C, the binding free energies (G = -RTlnKD) were -4.94 kcal $mol^{-1}$ for NCI668036 and -6.82 kcal $mol^{-1}$ for the Dapper peptide.

**Inhibition of the Wnt Signaling Pathway By NCI668036**

In an earlier study, it was demonstrated that the PDZ domain of Dvl interacts directly with the conserved sequence that is C terminal to the seventh transmembrane helix of the Wnt receptor Fz. This interaction is essential in transduction of the Wnt signal from Fz to the downstream component of Dvl. Therefore, an inhibitor of the Dvl PDZ domain should modulate Wnt signaling by acting as an antagonist. To test whether NCI668036 can indeed inhibit Wnt signaling pathways, NCI668036 was co-injected with various activators of the canonical Wnt pathway into the animal-pole region of *Xenopus* embryos at the two-cell stage. RT-PCR was the performed to analyze expression of the Wnt target gene *Siamois* in ectodermal explants that were dissected from blastulae and cultured until their development reached the early gastrula stage. In the RT-PCR experiments, expression of ornithine decarboxylase (ODC) was used as the loading control. Although NCI668036 had little effect on *Siamois* expression induced by $\beta$-catenin, a component of Wnt signaling that is downstream of Dvl, NCI668036 inhibited *Siamois* expression induced by Wnt3A (Fig. 7A). These results are consistent with the notion that binding of NCI668036 to the PDZ domain of Dvl blocks signaling in the canonical Wnt pathway at the Dvl level.

Whether NCI668036 affected the well-known ability of Wnt to induce secondary axis formation was then tested. Wnt3A injected into the ventro-vegetal region of a *Xenopus* ectodermal explant induced the formation of a complete secondary $axis_{37}$ (Fig. 7B and 7C). However, when co-injected with Wnt3A, NCI668036 substantially reduced the secondary axis formation induced by Wnt3A (Fig. 7D). This reduction resulted in embryos with a partial secondary axis or only a single axis (see Table 6). Therefore, it may be concluded that NCI668036 specifically blocks signaling in the canonical Wnt pathway.

By using a UNITY™ search for compounds with the potential to bind to the PDZ domain, FlexX™ docking of candidates into the binding site, Cscore™ ranking of binding modes, and chemical-shift perturbation NMR experiments, we identified a non peptidic small organic molecule (NCI668036) was identified, which could bind to the mDvlI PDZ domain. This shows that NMR-assisted virtual ligand screening is a feasible approach to identify small molecules that, on the basis of their structural features, are predicted to bind to the target

To build the search query for the virtual-screening stage, the crystal structure of the PDZ domain of *Xenopus* Dvl bound with the Dapper peptide was used instead of the NMR solution structure of the apo-PDZ domain of mouse Dvl. The two PDZ domains share high homology, especially around the peptide-binding sites; near the binding sites; there is only a single amino acid difference between the two PDZ domains (Glu319 in the PDZ domain of mDvl1 versus Asp326 in the PDZ domain of *Xenopus* Dvl), and the side chain of this residue points away from the peptide-binding cleft. The peptide-binding cavity of the domain is smaller in the apo-form of the solution structure than in the crystal structure of the Dapper-bound PDZ domain of *Xenopus* Dv1. This difference is consistent with the classic "induce-and-fit" mechanism, in which, upon the binding of a peptide or a small organic molecule, the binding sites in the PDZ domain undergo conformational change to accommodate the bound ligand. However, this flexibility cannot be fully explored through UNITY™ search and the FlexX™ docking protocols. Therefore, although the PDZ domain of mouse Dvl was used in the experimental studies, the crystal structure of the PDZ domain of *Xenopus* Dvl provides a better template for the virtual screening steps. Indeed, the binding free energies calculated from MD simulation of the PDZ domain-NCI668036 and PDZ domain-Dapper peptide complexes fit well with the experimental binding data.

NCI668036 is a peptide mimetic in which two peptide bonds are substituted by two ether bonds. Therefore NCI668036 is expected to be more stable than the corresponding peptide in vivo. Although it binds the PDZ domain relatively weakly, NCI668036 can be used as a template for further modifications. Indeed, NCI668036 has a very simple structure, and it is very stable and highly soluble. In addiction, MD simulation showed that, compared with the complex of the PDZ domain and Dapper peptide, which has higher binding affinity (Kd = 10 μM), the complex formed by the PDZ domain and NCI668036 does not fully utilize all possible interactions to maximize binding affinity. For example, the binding affinity is expected to increase if the branching of a hydrophobic group from the backbone of NCI668036 contacts the side chain of Phe257 in the PDZ domain.

NCI668036 interacts with the Dvl PDZ domain specifically. We tested two other PDZ domains: the first PDZ domain of PSD-95, PSD95a (PDB code: 1IU0, 1IU2), which belongs to the class I PDZ domains, and the PDZ7 domain of the glutamate receptor-interacting protein (PDB code: 1M5Z), a member of class II PDZ domains (Fig. 12 shows the structure-based sequence alignment of different PDZ domains). NCI668036 binds to both of these PDZ domains extremely weakly. The specificity of NCI668036 for the Dvl PDZ domain likely is due to a unique feature of the domain. The Dvl PDZ domain belongs to neither class I nor class II PDZ domains. (Fig. 12). In particular, the Dvl PDZ domain has two loops: one is between the first and second β-strands (the βA-βB loop), and the other is between the second α-helix and the last β-strand (the βB-βF loop). These two loops of the Dvl PDZ domain are longer than that in a typical PDZ domain. In the structure of a typical PDZ domain bound with a C-terminal peptide, the carboxylate group of the bound peptide is also linked through a bound water molecule to the guanidinium group of an arginine in the βA-βB loop. The side chain of the same arginine also forms a hydrogen bond with the amide ground of a glycine in the βB-βF loop. However, the Dvl PDZ domain lacks both the arginine and glycine, and the cavity that holds the bound water molecule in a typical PDZ domain is much smaller in the Dvl PDZ. Indeed, there is no bound water molecule in the crystal structure of the Dvl PDZ domain in a complex with the Dapper peptide. However, when NCI668036 bound to the Dvl PDZ domain, oxygen 03 participated in two hydrogen-bond connections with the "carboxylate binding loop" of the PDZ domain, and the carboxylate group of the bound NCI668036 was pushed into the empty space and stayed in the narrow cavity. We speculate that this binding feature of NCI668036 may explain the specificity of the molecule for the Dvl PDZ domain; in other words, NCI668036 achieves its specificity by using its unique binding mode. This notion is supported by results from one of our MD simulation studies. In the MD simulation run, the starting conformation of the PDZ domain. NCI668036 complex was created by superimposing NCI668036 over the bound Dapper peptide, so that the carboxylate group of the compound formed all three hydrogen bonds with the host PDZ domain. After a 200-ps production run, the system was no longer stable.

Using the screening methods described, additional compounds were identified which were found to bind to a domain of the Dishevelled proteins. Fig. 8 shows the structures of molecular compounds which were all found capable of binding to the Dishevelled proteins. Fig. 9 and Fig. 10 show structures of compounds that bind to Dishevelled, and they also show compounds which were found to be non-binding. All of the compound structures in Fig. 11 were found to bind to the PDZ domain of the Dishevelled protein. These compounds were NCI compounds, Sigma Aldrich compounds and Chem Div compounds.

Considering that Dvl is at the crossroad of the Wnt signaling pathways and that the typical binding events in which the molecule is involved are relatively weak but finely tuned and well balanced, an effective Dvl antagonist might be very useful in analyses of Wnt signaling and in dissecting various pathways. Functional studies of NCI668036 strongly support this theory. Besides being a powerful tool for biological studies of Wnt signaling pathways, a strong inhibitor of Dvl serves as a leading compound for further development of pharmaceutical agents useful in the treatment of cancer and other human diseases in which the Wnt signaling pathways has a crucial role in pathogenesis.

MATERIALS AND METHODS

**Purification of $^{15}$N-labeled mDvl1 PDZ Doimain.**

The $^{15}$N-labeled mouse Dvl1 PDZ domain (residue 247 to residue. 341 of mDvl1) was prepared as described previously. To increase the solubility of the protein, Cys334, which is located outside the ligand binding site, was mutated to alanine in the PDZ domain construct

**Preparation of 2-((5(6)-Tetramethylrhodamine)carboxylamino)ethyl Methanethiosulfonate (TMR)-Linked mDvl1 PDZ Domain.**

Wild-type PDZ domain protein (without the Cys334Ala mutation) was produced using the standard procedure. Cys334 is the only cysteine in the protein. Purified PDZ (40 μM) was dialyzed against 100 mM potassium phosphate buffer (pH 7.5) at 4 °C overnight to remove DTT, which was added during protein purification steps to prevent disulfide bond formation. We then dropwise added a 10-fold molar excess of TMR dissolved in DMSO to the solution of the PDZ domain while it was being stirred. After 2 hours of reaction at room temperature, excess TMR and other reactants were removed by extensive dialysis against 100 mM potassium phosphate bufferpH 7.5) at 4 °C.

**Structure-based Ligand Screening of Small Compounds Binding to the PDZ Domain.**

The UNITY™ module of the SYBYL™ software package (Tripos, Inc.) was used to screen the NCI small-molecule 3D database for chemical compounds that could fit into the peptide-binding groove of the Dvl PDZ domain (PDB code: 1L60). The candidate compounds then were docked into the binding groove by using the FlexX™ module of SYBYL™ (Tripos, Inc.). The compounds that displayed the highest consensus binding scores were acquired from the Drug Synthesis and Chemistry Branch, Developmental Therapeutics Program, Division of Cancer Treatment and Diagnosis, National Cancer Institute (http://129.43.27.140/ncidb2/) for further tests.

**NMR Spectroscopy.**

NMR $^{15}$N-HSQC experiments were performed by using a Varian Inova 600-MHz NMR spectrometer at 25 °C. Samples consisted of the Dvl PDZ domain (concentration, -0.3 mM) in 100 mM potassium phosphate buffer (pH 7.5), 10% $D_2O$, and 0.5 mM EDTA. NMR spectra were processed with NMRpipe software and analyzed by using the program Sparky™.

**Fluorescence Spectroscopy.**

We used a Fluorolog-3 spectrofluorometer (Jobia-Yvon, Inc.) was used to obtain the fluorescence measurements of the interaction between the TMP-linked PDZ domain, and the NCI668036 compound. Titration experiments were performed at 25 °C in 100 mM potassium phosphate buffer (pH 7.5). The solution of NCI668036 (concentration, 1 mM) was sequentially injected into a fluorescence sample cell that contained 2 ml 30 μM TMR-labeled PDZ domain in 100 mM potassium phosphate buffer (pH 7.5). During the fluorescence measurement, the excitation wavelength was 552 nm, and the emission wavelength was 579 nm. The fluorescence data were analyzed by using the ORIGIN™ program (Microcal Software, Inc.). The $K_D$ values were determined by using a double reciprocal plot of fluorescence changes against increasing compound concentrations.

**Molecular Dynamics Simulation.**

MD simulation was performed by using the sander program in the software package AMBER 8™ with the parm99 force field. AM1-BCC charges were assigned to NCI668036 by using the Antechamber module 47 in AMBER 8. ™ The starting structures of ligand-protein complexes were prepared by using the output from the FlexX™ docking studies. After neutralization, complexes were dissolved in a periodic rectangular TIP3P water box, with each side 10 A away from the edge of the system. The components of these MD systems are summarized in Table 1 Systems were minimized by 1000-step steepest. descent minimization followed by 9000-step conjugated gradient minimization. The MD simulations were performed with time step of 2 ps and non-bonded cutoff being set to 9.0 Å. Both constant volume (NTV) and constant pressure (NTP) periodic boundary conditions were applied to gradually relax the system. In detail, the MD production run was carried out under the NPT condition for 5 ns after a 50-ps NVT ensemble in which the temperature was increased from 100 K to 300 K, a 50-ps NPT ensemble in which solvent density was adjusted, and another 100-ps NPT ensemble in which harmonic restraints were gradually reduced from 5.0 kcal mol-1 Å-2 to 0. Snapshots were saved every 5 ps during the production run. Other simulation parameters were set similarly to those described in the work by Gohlke et al.

**Binding Free Energy Calculation.**

Binding free energy was calculated by (1) for which the MM-PBSA approach was implemented by using the mm_pbsa.p1 module of AMBER 8™.

$$G^{total} = G^{complex} - G^{protein} - G^{ligand} \quad (1)$$

were

$$G = H_{gas} + H_{transrot} + G_{solvation} - TS \quad (2)$$

$$G_{solvation} = G_{polar\ solvation} + G_{nonpolar\ solvation} \quad (3)$$

$$G_{nonpolar\ solvation} = yA + b \quad (4)$$

Where gas phase energy, $H_{gas}$, is the sum of internal (bond, angle, and torsion), van der Waals, and electrostatic energy in the molecular mechanical force field with no cutoff, as calculated by molecular mechanics. Htrans/rot is 3RT (R is the gas constant) because of six translational and rotational degrees of freedom. Solvation free energy, $G_{solvation}$, was calculated by using the PB model. In PB calculations, the polar salvation energy, $G_{polar\ solvation}$, was obtained by solving the PD equation by with the Delphi software using parse radius, parm94 charges (for the PDZ domain and the Dapper peptide), and AM1-BCC charges (for the compound). The nonpolar contribution was calculated by (4). In the equation, A is the solvent accessible area calculated by the Molsurf module in Amber 8™, and *y* (surface tension) and b (a constant) were 0.00542 kcal $mol^{-1}$, Å-2 and 0.92 kcal $mol^{-1}$ respectively. All of the above energy terms were averaged from 150 snapshots extracted every 20 ps, and entropy TS was estimated by normal mode analyses using 15 snapshots extracted every 200 ps during the last 3-ns production run.

DETAILED DESCRIPTION OF THE FIGURES

**Figure 1. Structure of compound NCI668036.**
The chemical structure of NCI668036 was sketched by using ISIS/Draw (MDL Information Systems, Inc.). Some atoms (which are mentioned previously) are labeled with the atom name assigned by the Antechamber module of AMBER 8™.

**Figure 2. Interaction between the mDvl1 PDZ domain and NCI668036.**
$^{15}N$-HSQC spectra of free NCI668036 (red contour lines) and of NCI668036 bound to the PDZ domain of mDvl1 (blue contour lines) are shown. The concentration of the PDZ domain was 0.3 mM. The concentrations of NCI668036 was 7.8 mM (bound form). In the upper inset, the signals from the same region witch enlarged spectra were placed in smaller boxes. The inset also contains an additional spectrum (green lines) from a different concentration of NCI668036 (2.4 mM). In the worm representation of the backbone structure of the mDvl1 PDZ domain (lower inset), the thickness of the worm is proportional to the weighted sum (in Hz) of the $^{1}H$ and $^{15}N$ shifts upon binding by NCI668036; increasing chemical-shift perturbation is shown (blue, low; red, high). The figure was prepared by using the software Insight II™ (Accelrys, Inc.).

**Figure 3. Binding affinity between mDvl1 PDZ and NCI668036 as determined from a double reciprocal plot of fluorescence intensity quenching (F) against the concentration of NCI668036.**
Fluorescence measurements were obtained by titrating NCI668036 into a solution of the TMR-PDZ domain. The $K_D$ value of the complex formed by NCI668036 and the PDZ domain of mDvl1 was 237 $\pm$ 31 $\mu$M as extracted after linear fitting.

**Figure 4. The 30 docking conformations of compound NCI668036 generated by using the FlexX™ program were clustered into three groups.**
Group I comprised 5 conformations (red) with RMSDs between 0.46 and 0.77 Å, group II had 13 conformations (yellow) with RMSDs between 1.44 and 1.73 Å, and group III had 12 conformations (blue) with RMSDs between 2.31 and 2.86 Å.

**Figure 5. Backbone root mean square deviations (RMSDs, Å) of the mDvl1 PDZ domain bound to NCI668036, the mDvl1 PDZ domain bound to the Dapper peptide, and the starting structure and total potential energies of the MD systems for 5-ns explicit simulations.**
The 200-ps equilibration phase is not included.

A. Backbone RMSDs of the mDvl1 PDZ domain (purple) and NCI668036 (green) for a 5-ns simulation.
B. Backbone RMSDs of the Dvl1 PDZ domain (purple) and Dapper peptide (green) for a 5-ns simulation.
C. The total potential energy (ETOT) of the mDvl1 PDZ domain and NCI668036 (water molecules included) during a 5-ns simulation fluctuated between -44552.6 kcal $mol^{-1}$ and - 44344.2 kcal $mol^{-1}$. The total potential

energy (mean ± standard deviation) was -44450.8 ± 32.6 kcal $mol^{-1}$.

D. The total potential energy of the Dvl PDZ domain (water molecules included) and Dapper peptide during a 5-ns simulation fluctuated between -44349.8 kcal $mol^{-1}$ and -44122.3 kcal $mol^{-1}$. The total potential energy (mean ± standard deviation) was -44233.8 ± 31.3 kcal $mol^{-1}$.

**Figure 6. Conformation of NCI668036 docked into the PDZ domain and of the NCI668036-mDvl1 PDZ domain complex.**

A. NCI668036 and the Dapper peptide bound to the PDZ domain in similar conformations. NCI668036 (blue) was docked into the Dvl PDZ domain (ribbons and tubes in gray) by using FlexX™ (Tripos, Inc.). The Dapper peptide (orange) is in its conformation determine by x-ray crystallography and is in a complex with the PDZ domain. The difference between the backbone root mean square deviation of compound NCI668036 and that of Dapper peptide (only the 4 C-terminal amino acids [MTTV] backbone atoms were used) was 1.49 Å.

B. The binding conformation of NCI668036 at 4.905 ns during the 5-ns simulation. The PDZ domain is shown as gray ribbons and tubes. NCI668036 is represented according to the bound atom (green, carbon; red, oxygen and blue, nitrogen). Residues that formed a hydrogen bond with the compound are shown in ball-and-stick format (black, carbon; red, oxygen; blue, nitrogen); hydrogen bonds are represented by yellow dashed lines. Residues within 3.5 A of isopropyl, methyl (those next to nitrogen atoms), and *t*-butyl groups of compound are in CPK format (gray, carbon; red, oxygen; blue, nitrogen. In addition, Leu258, Ile260, and Ile262 were within 3.5 Å of the isopropyl group next to the carboxylate group. They are in ball-and-stick format for clarity.

**Figure 7. Effect of NCI668036 on canonical Wnt signaling in *Xenopus* embryos.**

A. NCI668036 inhibited the canonical Wnt pathway induced by Wnt3A but not by β-catenin. RT-PCR was conducted to analyze the expression of the *Xenopus* Wnt target gene *Siamois* in ectodermal explants. Synthetic mRNA corresponding to Wnt3A (1 pg) and â-catenin (500 ng) were injected alone or with NCI668036 (180 ng) into the animal-pole region at the two-cell stage, and ectodermal explants were cultured until they reached the early gastrula stage, at which time they underwent RT-PCR analysis.

B. A control embryo that received no injection.

C. An embryo that received an injection of Wnt3A mRNA developed a complete secondary axis.

D. An embryo that received coinjections of Wnt3A mRNA and NCI668036 developed a partial secondary axis.

**Figure 8. Molecular structures of NCI & Sigma Aldrich compounds which were tested for their ability to bind to the Dishevelled protein.**

Compounds 221120, 107146, 145882 and 161613 were found to weakly bind to Dvl whereas compounds 108123, 339938, v8878 and 579270 were found to not bind at all.

**Figure 9. Molecular structures of Chem Div compounds which were tested for their ability to bind to the Dishevelled protein.**

Compounds 3237-0565, 3237-0713, 3237-0430, 8006-2560, 0090-0031 and 2372-2393 were found to bind to Dvl whereas 0136-0181 did not.

**Figure 10. Molecular structures of Chem Div compounds which were tested for their ability to bind to the Dishevelled protein.**

Compounds 8004-1312, 3289-8625, 3289-5066, 3237-0719 bound to Dvl. Compounds 8003-2178, C691-0030, 1748-0253, 1108-0424, 2922-0102, 3379-2274 and 8003-4726 did not bind to Dvl.

**Figure 11. Molecular structures of compounds which were tested for their ability to bind to the Dishevelled protein.**

Compounds 103673, 145882, 3289-5066, 3289-8625, 337837, 7129, 3237-0719, 12517, p1, 142277, 82569, 39869, p3, 46893, 661075, 661080, 661086, 661092, 661091, 84123 and 668036 were all found to bind to Dvl.

**Figure 12. Structure-based alignment of the amino-acid sequences of the PDZ domains of Dvl Homologs and other proteins.**

Secondary structural elements are indicated above the sequences. Residues at the gly-his (GH) positions are in boldface type. The asterisk denotes the binding pocket for the ligand's C terminus. Sequence differences among the PDZ domains are indicated by underlining.

**Table 1.** Information about atoms of simulated systems and dimensions of water boxes.

**Table 2.** Binding free energy components of compound NCI668036 and PDZ averaged over the last 3 ns of a 5-ns explicit simulation.

**Table 3.** Binding free energy components of the PDZ domain and the Dapper peptide averaged over the last 3 ns of a 5-ns explicit simulation.

**Table 4.** Binding free energy components of the PDZ domain and NCI668036 and the PDZ domain and the Dapper peptide averaged over the last 3 ns of the 5-ns explicit simulation.

**Table 5.** Hydrogen bonds observed between NCI668036 and the PDZ domain and between the Dapper peptide and the PDZ domain during 5-ns explicit simulation.

**Table 6.** Effect of NCI668036 on formation of the secondary axis induced by Wnt3A and ß-catenin.

[a]Ventro-vegetal injection of Wnt3A mRNA and β-catenin and of Wnt3A mRNA and NCI668036 at the two-cell stage. Experimental details are shown in Figure 7B through D.
[b]Defined as the appearance of a second neural plate on the ventral side of early neurulae and ectopic eyes and cement glands. Percentages indicate the proportion of embryos that met the definition.
[c]Total number of embryos that received injections in two independent experiments.

Table 1: Atom information of simulated systems and dimensions of water boxes

| Complex | PDZ-NCI668036 | PDZ-Dapper peptide |
|---|---|---|
| No. of atoms in the ligand | 67 | 135 |
| No. of residues in the ligand | 1 | 8 |
| No. of atoms in the protein | 1348 | 1348 |
| No. of residues in the protein | 90 | 90 |
| No. of Na+ atoms | 5 | 3 |
| No. of TIP3P molecules | 5399 | 5372 |
| Total no. of atoms | 17617 | 17602 |
| Box size | 62Å×67Å×56Å | 62Å×67Å×56Å |

Table 2: Binding free energy components of compound NCI668036 and PDZ averaged over the last 3 ns of 5 ns explicitly simulation[a]

| | PDZ-NCI668036 | | | PDZ | | | NCI668036 | | | Delta[b] | |
|---|---|---|---|---|---|---|---|---|---|---|---|
| Contrib.[c] | Mean[d] | SE[e] | | Mean[d] | SEd[e] | | Mean[d] | SE[e] | | Mean[d] | SE[e] |
| $H_{elec}$ | -2726.05 | 49.15 | | -2738.88 | 52.64 | | 7.31 | 2.69 | | 5.52 | 12.57 |
| $H_{vdw}$ | -306.94 | 15.67 | | -272.72 | 14.71 | | 6.18 | 2.69 | | -40.39 | 2.84 |
| $H_{int}$ | 1832.79 | 27.16 | | 1760.28 | 25.7 | | 72.51 | 5.87 | | 0 | 0 |
| $H_{gas}$ | -1200.2 | 56.31 | | -1251.32 | 59.51 | | 86 | 6.13 | | -34.88 | 12.93 |
| $PB_{sur}$ | 31.8 | 0.5 | | 31.9 | 0.5 | | 5.17 | 0.06 | | -5.27 | 0.16 |
| $PB_{cal}$ | -1777.12 | 47.65 | | -1675.18 | 51.38 | | -118.57 | 2.4 | | 16.63 | 12.78 |
| $PB_{sol}$ | -1745.32 | 47.41 | | -1643.28 | 51.13 | | -113.4 | 2.42 | | 11.36 | 12.71 |
| $PB_{tot}$ | -2945.52 | 27.48 | | -28.94.6 | 27.13 | | -27.4 | 5.38 | | -23.52 | 3.36 |
| $TS_{tra}$ | 16.03 | 0 | | 15.99 | 0 | | 13.27 | 0 | | -13.23 | 0 |
| $TS_{rot}$ | 15.83 | 0.01 | | 15.79 | 0.01 | | 11.3 | 0.21 | | -11.25 | 0.2 |

(continued)

| | PDZ-NCI668036 | | | PDZ | | | NCI668036 | | | Delta[b] | |
|---|---|---|---|---|---|---|---|---|---|---|---|
| Contrib.[c] | Mean[d] | SE[e] | | Mean[d] | SEd[e] | | Mean[d] | SE[e] | | Mean[d] | SE[e] |
| $TS_{vib}$ | 1022.07 | 4.96 | | 973.56 | 4.65 | | 45.67 | 1.62 | | 2.84 | 4.96 |
| $TS_{tot}$ | 1053.93 | 4.96 | | 1005.34 | 4.65 | | 70.24 | 1.83 | | -21.64 | 5.02 |
| $\Delta G_{total}$ | | | | | | | | | | -1.88 | |

[a]All energies in kcal $mol^{-1}$.
[b]Contribution (PDZ-NCI668036) - Contribution (PDZ) - Contribution (NCI668036).
[c]$H_{elec}$, coulombic energy; $H_{vdw}$, van der Waals energy; $H_{int}$, internal energy; $H_{gas} = H_{elec} + H_{vdw} + H_{int}$; $PB_{sur}$, non-polar contribution for solvation free energy; $PB_{cal}$, polar contribution fro salvation free energy; $PB_{sol} = PB_{sur} + PB_{cal}$; $PB_{tot} = H_{gas} + PB_{sol}$; $TS_{tra}$/ $TS_{rol}$/ $TS_{vib}$, translational/rotational/vibrational entropy; $TS_{tot} = TS_{tra} + TS_{rot} + TS_{vib}$; $\Delta G_{total} = PB_{tot} + H_{trans/rot} - TS_{tot}$.
[d]Average over 150 snapshots and 15 snapshots for entropy contributions.
[e]Standard error of mean values.

Table 3: Binding free energy components of the PDZ domain and Dapper peptide averaged over the last 3ns of 5 ns explicitly simulation[a]

| | PDZ-Dapper peptide | | | PDZ | | | Dapper peptide | | | Delta | |
|---|---|---|---|---|---|---|---|---|---|---|---|
| | Mean | Std | | Mean | Std | | Mean | Std | | Mean | Std |
| $H_{elec}$ | -3076.24 | 56.04 | | -2759.74 | 50.83 | | -127.92 | 10.73 | | -188.58 | 22.76 |
| $H_{vdw}$ | -315.8 | 17.33 | | -268.01 | 16.27 | | 5.66 | 3.81 | | -53.46 | 3.51 |
| $H_{int}$ | 1926.1 | 25.44 | | 1774.73 | 25.03 | | 151.37 | 7.34 | | 0 | 0 |
| $H_{gas}$ | -1465.94 | 57.68 | | -1253.02 | 51.63 | | 29.11 | 12.13 | | -242.03 | 23.04 |
| $PB_{sur}$ | 34.03 | 0.6 | | 32.83 | 0.57 | | 8.21 | 0.18 | | -7.02 | 0.18 |
| $PB_{cat}$ | -1764.06 | 55.33 | | -1660.76 | 47.57 | | -318.15 | 10.32 | | 214.85 | 22.79 |
| $PB_{sol}$ | -1730.03 | 55.1 | | -1627.93 | 47.34 | | -309.94 | 10.3 | | 207.83 | 22.73 |
| $PB_{tot}$ | -3195.97 | 25.91 | | -2880.94 | 25.17 | | -280.83 | 7.24 | | -34.2 | 4.13 |
| $TS_{tra}$ | 16.07 | 0 | | 15.99 | 0 | | 13.86 | 0 | | -13.78 | 0 |
| $TS_{rot}$ | 15.9 | 0.02 | | 15.79 | 0.01 | | 12.54 | 0.05 | | -12.42 | 0.05 |
| $TS_{vib}$ | 1069.73 | 5.22 | | 969.69 | 3.62 | | 100.55 | 0.69 | | -0.51 | 6.37 |
| $TS_{tot}$ | 1101.7 | 5.23 | | 1001.47 | 3.63 | | 126.95 | 0.71 | | -26.72 | 6.37. |
| $\Delta G_{total}$ | | | | | | | | | | -7.48 | |

[a]Abbreviations and equations are the same as those defined for Supplemental Table 2.

Table 4: Binding free energy components of the PDZ domain and NCI668036, the PDZ and Dapper peptide averaged over the last 3 ns of 5 ns explicitly simulation[a]

| Contrib.[b] | $\Delta H_{elec}$ | $\Delta H_{vdw}$ | $\Delta H_{gas}$ | $\Delta PB_{cal}$ | $\Delta PB_{sur}$ | $\Delta PB_{sol}$ | $\Delta PB_{tot}$ | $T\Delta S$ | $\Delta G_{total}$ |
|---|---|---|---|---|---|---|---|---|---|
| NCI668036 | 5.52 | -40.39 | 0 | 16.63 | -5.27 | 11.36 | -23.52 | -21.64 | -1.88 |

(continued)

| Contrib.[b] | $\Delta H_{elec}$ | $\Delta H_{vdw}$ | $\Delta H_{gas}$ | $\Delta PB_{cal}$ | $\Delta PB_{sur}$ | $\Delta PB_{sol}$ | $\Delta PB_{tot}$ | $T\Delta S$ | $\Delta G_{total}$ |
|---|---|---|---|---|---|---|---|---|---|
| Dapper peptide | -188.58 | -53.46 | 0 | 214.85 | -7.02 | 207.83 | -34.20 | -26.72 | -7.48 |

[a]All energies are in kcal $mol^{-1}$.
[b]Contribution (PDZ-NCI668036) - Contribution (PDZ) - Contribution (NCI668036) for NCI668036 and Contribution (PDZ-Dapper peptide) - Contribution (PDZ) - Contribution (Dapper peptide) for Dapper peptide. $H_{elec}$, coulomic energy; $H_{vdw}$, van der Waals energy; $H_{int}$, internal energy; $\Delta H_{gas} = \Delta H_{elec} + \Delta H_{vdw} + \Delta H_{int}$; $PB_{sur}$, non-polar contribution for solvation free energy; $PB_{cal}$, polar contribution for solvation free energy; $\Delta PB_{sol} = \Delta PB_{sur} + \Delta PB_{cal}$; $\Delta PB_{tot} = \Delta H_{gas} + \Delta PB_{sol}$; $T\Delta S = T\Delta S_{tra} + T\Delta S_{rot} + T\Delta S_{vib}$; $\Delta G_{total} = \Delta PB_{tot} + \Delta H_{trans/rot} - T\Delta S$.

Table 5: H-bonds observed between compound NCI668036 and PDZ, Dapper peptide and PDZ during 5 ns explicitly simulation[a]

| NCI668036 - PDZ | | | | Dapper peptide - PDZ | | |
|---|---|---|---|---|---|---|
| NCI668036 | PDZ | Occupancy[b] | | Dapper peptide | PDZ | Occupancy[b] |
| O | Leu258N/H | 13.5 | | Val0OXT | Leu258N/H | 27.7 |
| O1 | Leu258N/H | 85.1 | | Val0O | Leu258N/H | 98.0 |
| O3 | Gly259N/H | 91.6 | | Val0OXT | Gly259N/H | 98.4 |
| O3 | Ile260N/H | 32.6 | | Val0OXT | Ile260N/H | 82.3 |
| N/H2 | Ile260N/H | 99.8 | | Val0N/H | Ile260N/H | 99.1 |
| O6 | Ile262N/H | 99.5 | | Thr-2O | Ile262N/H | 99.8 |
| N1/H5 | Ile262O | 65.1 | | Met-3N/H | Ile262O | 99.2 |
| O | Arg318 | 11.2 | | | | |
| | | | | Lys-5O | Gly264N/H | 99.4 |
| | | | | Lys-5N/H | Gly264O | 88.9 |
| | | | | Ser-7O | Ser266N/H | 85.3 |

[a]The length and angle cutoffs for H-bond are 3.5 Å and 120° respectively.
[b]Occupancy is in the units of percentage.

Table 6 Effect of the compound NCI668036 on the formation of secondary axis induced by Wnt3A and ß-catenin[a]

| | Double axis[b] | Single axis | Total[c] |
|---|---|---|---|
| No injection | | 100% | 83 |
| Wnt3A | 77% | 23% | 75 |
| Wnt3A/NCI668306 | 55% | 45% | 78 |
| β-catenin | 51% | 49% | 78 |
| β-catenin/NCI668386 | 49% | 51% | 76 |

[a]Ventro-vegetal injections of Wnt3A mRNA and β-catenin, and NCI668036 at two cell stage. Experimental details are shown in Figures 7B-7D.
[b]Defined as the appearance of a second neural plate on the ventral side of early neurulae and ectopic eyes and cement glands. Percentages indicate the proportion of embryos that met the definition.
[c]Total number of embryos that received injections in two independent experiments

## Claims

1. A compound selected from the group consisting of NCI668036, NCI221120, NCI107146, NCI145882, NCI161613, 8004-1312, 3289-8625, 3289-5066, 3237-0719, 3237-0565, 3237-0713, 3237-0430, 8006-2560, 0090-0031, 2372-2393, 103673, 145882, 3289-5066, 3289-8625, 337837, 7129, 3237-0719, 125217, p1, 142277, 82569, 39869, p3, 46893, 661075, 661080, 661086, 661092, 661091, 84123 or 668036 as shown in Figures 8-11 for use in the treatment of a disease in a mammalian subject, wherein the disease is colorectal cancer, desmoid cancer, endometrial cancer, gastric cancer, hepatocellular cancer, ovarian cancer, pancreatic cancer, prostate cancer, thyroid cancer, uterine cancer, breast cancer, Burkitt's lymphoma, medulloblastoma, Wilms' tumor, neuroblastoma, hepatoblastoma, diabetes, hair loss, bone fracture, bone disease, bone injury, loss of bone mass, a disease of sweat glands, or a disease of mammary glands.

2. The compound for use of claim 1, wherein said treatment comprises administration of the compound by inhalation, oral, intravenous, intraperitoneal, intramuscular, parenteral, transdermal, intravaginal, intranasal, mucosal, sublingual, topical, rectal, or subcutaneous administration, or any combination thereof.

3. The compound for use of claim 1, wherein the compound is formulated as a tablet, pill, dragee, liquid, gel, capsule, syrup, slurry or suspension.

4. Use of the compound of claim 1 for the manufacture of a medicament for treatment of a disease of claim 1.

5. The use of claim 4, wherein said treatment comprises administration of the compound by inhalation, oral, intravenous, intraperitoneal, intramuscular, parenteral, transdermal, intravaginal, intranasal, mucosal, sublingual, topical, rectal, or subcutaneous administration, or any combination thereof.

6. The use of claim 4, wherein the compound is formulated as a tablet, pill, dragee, liquid, gel, capsule, syrup, slurry or suspension.

## Patentansprüche

1. Verbindung, ausgewählt aus der Gruppe bestehend aus NCI668036, NCI221120, NCI107146, NCI145882, NCI161613, 8004-1312, 3289-8625, 3289-5066, 3237-0719, 3237-0565, 3237-0713, 3237-0430, 8006-2560, 0090-0031, 2372-2393, 103673, 145882, 3289-5066, 3289-8625, 337837, 7129, 3237-0719, 125217, p1, 142277, 82569, 39869, p3, 46893, 661075, 661080, 661086, 661092, 661091, 84123 oder 668036 wie aus den Figuren 8-11 ersichtlich zur Verwendung für die Behandlung einer Krankheit in einem Säuger, wobei die Erkrankung colorectaler Krebs, Desmoidkrebs, Endometriumkrebs, Magenkrebs, Leberzellkrebs, Eierstockkrebs, Bauchspeicheldrüsenkrebs, Prostatakrebs, Schilddrüsenkrebs, Gebärmutterkrebs, Brustkrebs, Burkitt-Lymphom, Medulloblastom, Wilms-Tumor, Neuroblastom, Hepatoblastom, Diabetes, Haarausfall, Knochenbruch, eine Knochenerkrankung, eine Knochenverletzung, Verlust von Knochenmasse, eine Erkrankung der Schweißdrüsen oder eine Erkrankung der Brustdrüsen ist.

2. Verbindung zur Verwendung nach Anspruch 1, wobei die Behandlung die Verabreichung der Verbindung durch Inhalation, orale, intravenöse, intraperitoneale, intramuskuläre, parenterale, transdermale, intravaginale, intranasale, mucosale, sublinguale, topische, rektale oder subkutane Verabreichung oder eine Kombination davon umfasst.

3. Verbindung zur Verwendung nach Anspruch 1, wobei die Verbindung als Tablette, Pille, Dragee, Flüssigkeit, Gel, Kapsel, Sirup, Aufschlämmung oder Suspension formuliert ist.

4. Verwendung der Verbindung nach Anspruch 1 zur Herstellung eines Medikaments für die Behandlung einer Erkrankung nach Anspruch 1.

5. Verwendung nach Anspruch 4, wobei die Behandlung die Verabreichung der Verbindung durch Inhalation, orale, intravenöse, intraperitoneale, intramuskuläre, parenterale, transdermale, intravaginale, intranasale, mucosale, sublinguale, topische, rektale oder subkutane Verabreichung oder eine Kombination davon umfasst.

6. Verwendung nach Anspruch 4, wobei die Verbindung als Tablette, Pille, Dragee, Flüssigkeit, Gel, Kapsel, Sirup, Aufschlämmung oder Suspension formuliert ist.

**Revendications**

1. Composé choisi parmi le groupe consistant en NCI668036, NCI221120, NCI107146, NCI145882, NCI161613, 8004-1312, 3289-8625, 3289-5066, 3237-0719, 3237-0565, 3237-0713, 3237-0430, 8006-2560, 0090-0031, 2372-2393, 103673, 145882, 3289-5066, 3289-8625, 337837, 7129, 3237-0719, 125217, p1, 142277, 82569, 39869, p3, 46893, 661075, 661080, 661086, 661092, 661091, 84123 ou 668036 comme montrés dans les figures 8-11 pour une utilisation dans le traitement d'une maladie chez un sujet mammifère, où la maladie est un cancer colorectal, un cancer desmoïde, un cancer de l'endomètre, un cancer gastrique, un cancer hépatocellulaire, un cancer de l'ovaire, un cancer du pancréas, un cancer de la prostate, un cancer de la thyroïde, un cancer de l'utérus, un cancer du sein, un lymphome de Burkitt, un médulloblastome, une tumeur de Wilms, un neuroblastome, un hépatoblastome, un diabète, une perte de cheveux, une fracture osseuse, une maladie osseuse, une lésion osseuse, une perte de masse osseuse, une maladie des glandes sudoripares, ou une maladie des glandes mammaires.

2. Composé pour l'utilisation de la revendication 1, où ledit traitement comprend l'administration du composé par inhalation, administration orale, intraveineuse, intrapéritonéale, intramusculaire, parentérale, transdermale, intravaginale, intranasale, muqueuse, sublinguale, topique, rectale, ou sous cutanée, ou n'importe quelle combinaison de celles-ci.

3. Composé pour l'utilisation de la revendication 1, où le composé est formulé en comprimé, pilule, dragée, liquide, gel, gélule, sirop, solution visqueuse ou suspension.

4. Utilisation du composé de la revendication 1 pour la fabrication d'un médicament pour le traitement d'une maladie de la revendication 1.

5. Utilisation de la revendication 4, où ledit traitement comprend l'administration du composé par inhalation, administration orale, intraveineuse, intrapéritonéale, intramusculaire, parentérale, transdermale, intravaginale, intranasale, muqueuse, sublinguale, topique, rectale, ou sous cutanée, ou n'importe quelle combinaison de celles-ci.

6. Utilisation de la revendication 4, où le composé est formulé en comprimé, pilule, dragée, liquide, gel, gélule, sirop, solution visqueuse ou suspension.

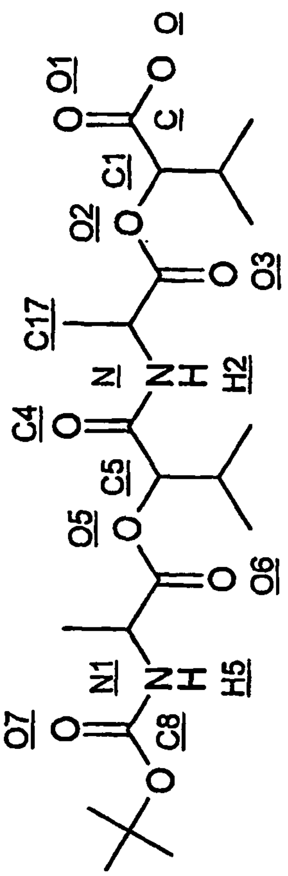

FIG. 1

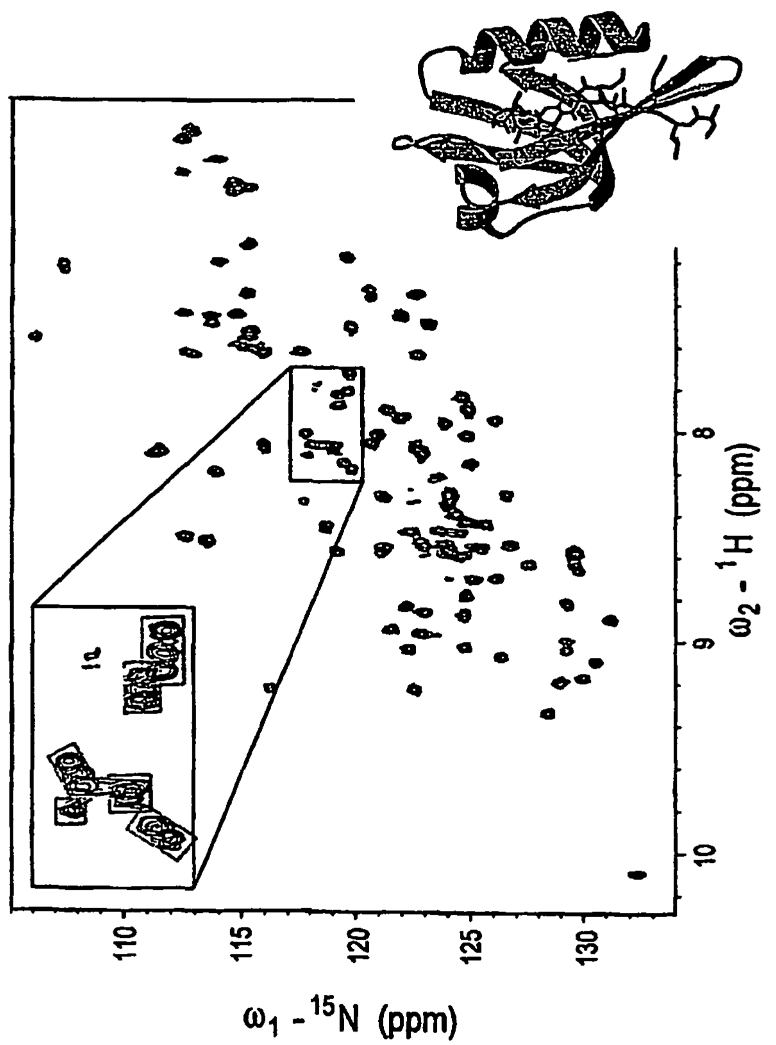


FIG. 2

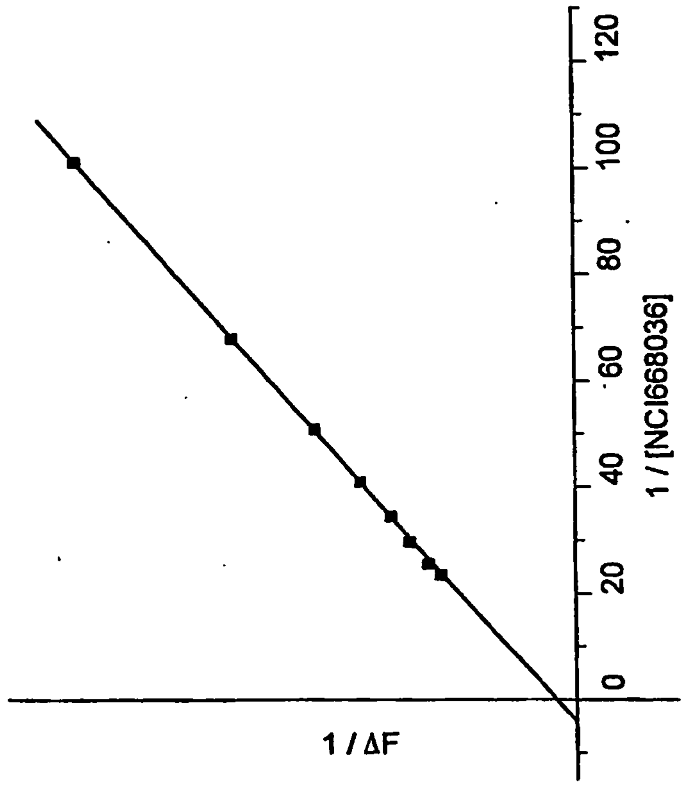


FIG. 3

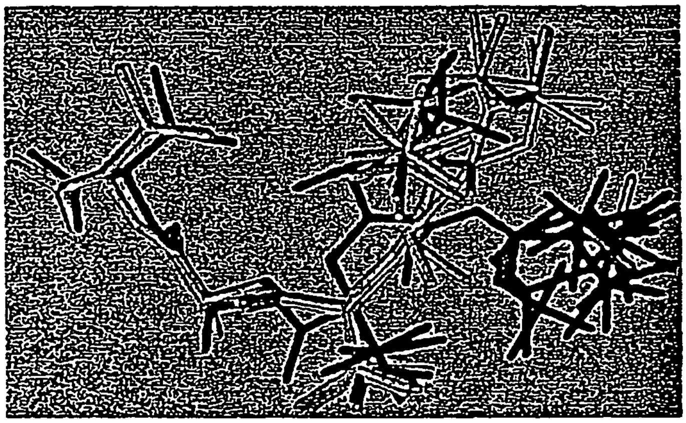

FIG. 4

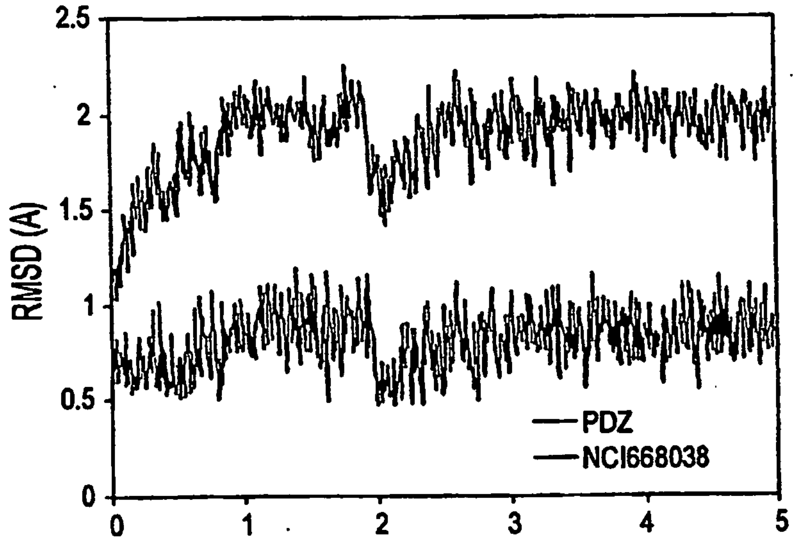


FIG. 5A

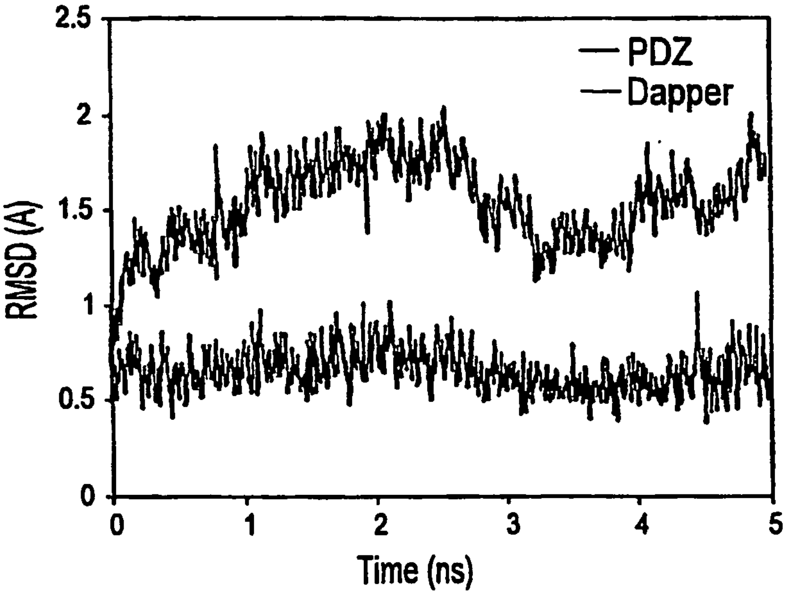


FIG. 5B

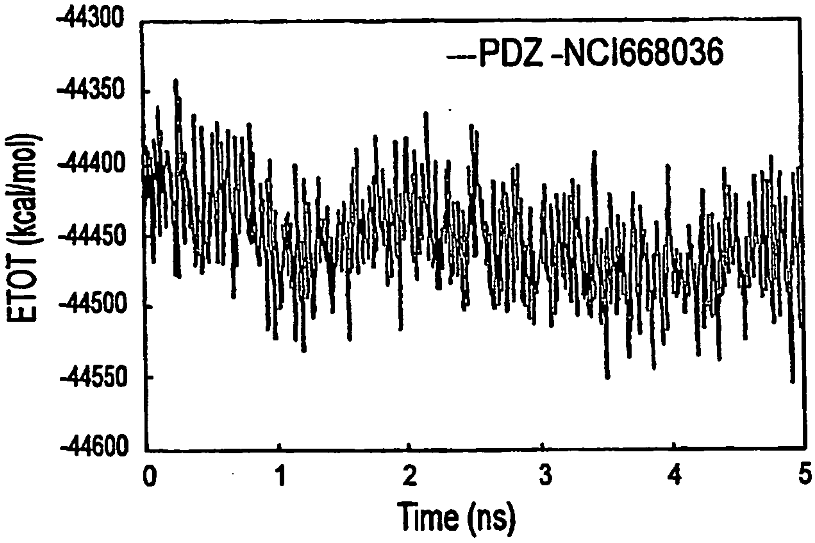

-44300
-44350
-44400
-44450
-44500
-44550
-44600
ETOT (kcal/mol)
—PDZ -NCI668036
0
1
2
3
4
5
Time (ns)


*FIG. 5C*

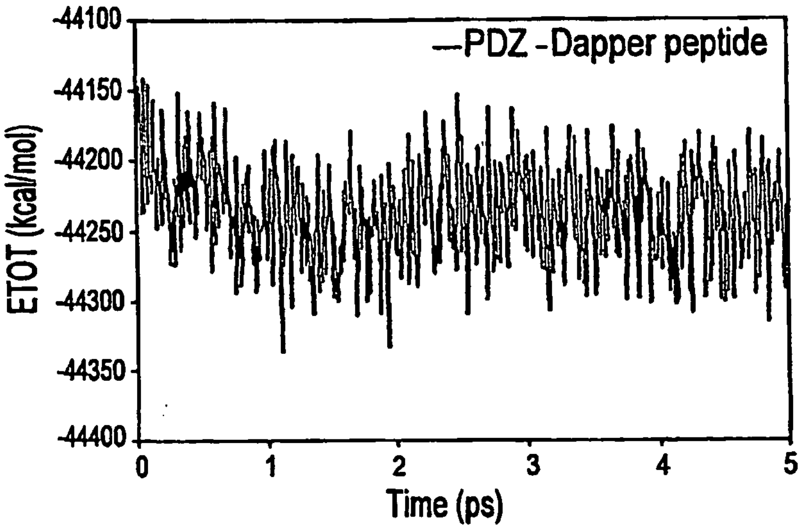
-44100
-44150
-44200
-44250
-44300
-44350
-44400
ETOT (kcal/mol)
—PDZ -Dapper peptide
0
1
2
3
4
5
Time (ps)

FIG. 5D

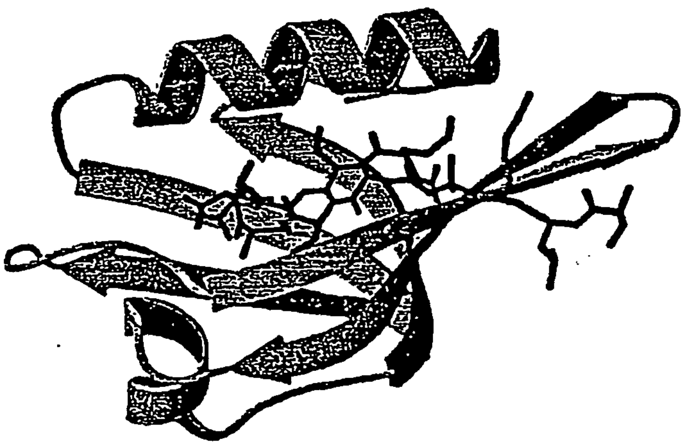

FIG. 6A

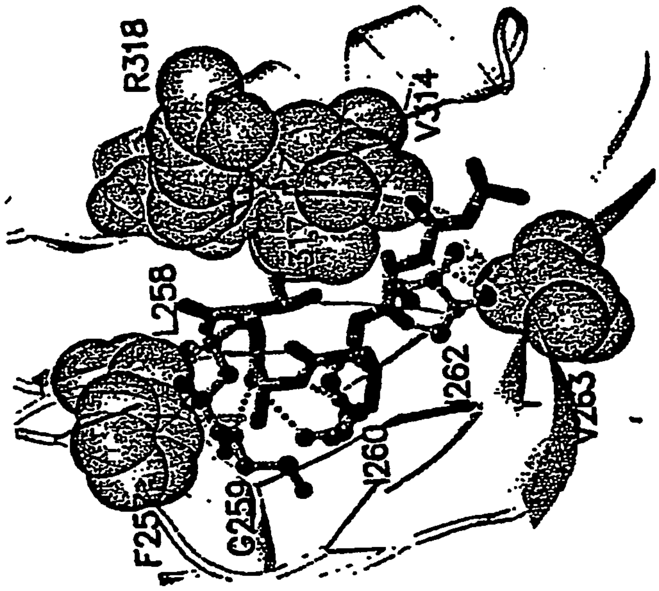


FIG. 6B

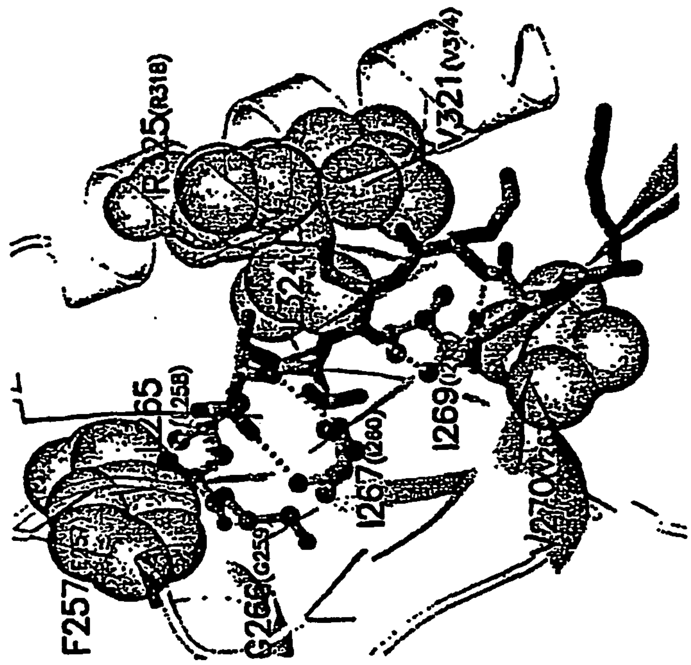


FIG. 6C

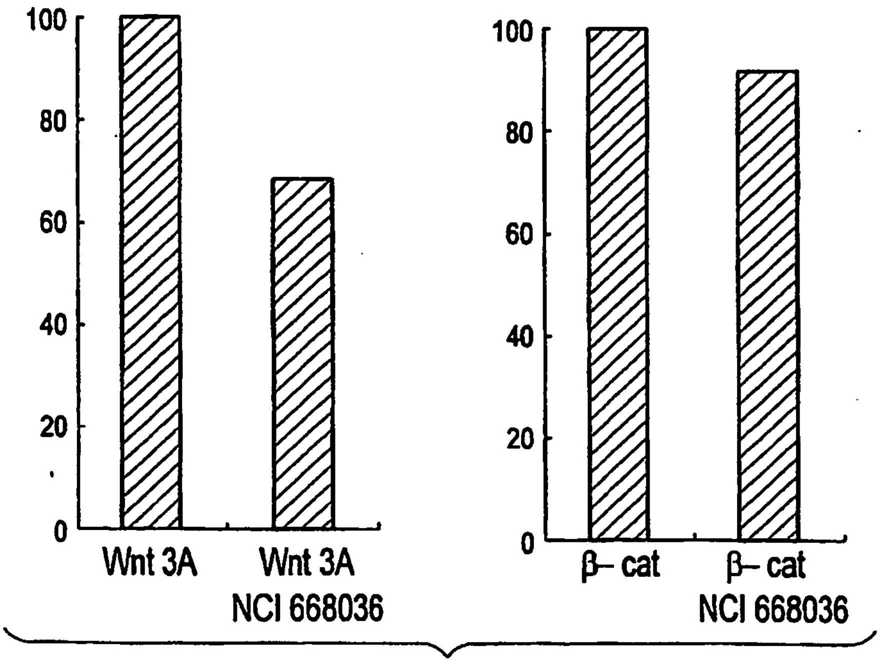
100
80
60
40
20
0
Wnt 3A
Wnt 3A
NCI 668036
100
80
60
40
20
0
β- cat
β- cat
NCI 668036

FIG. 7A

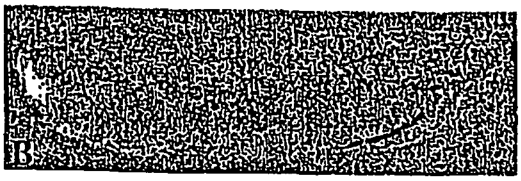

FIG. 7B

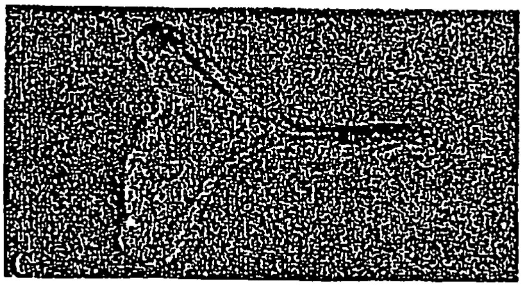

FIG. 7C

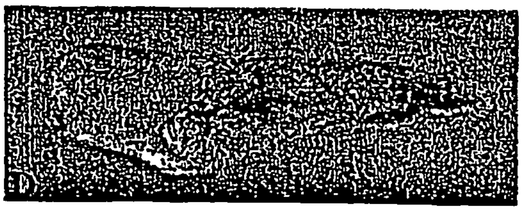

FIG. 7D

NCI & SIGMA ALDRICH COMPOUNDS

| WEAK BINDING COMPOUNDS | | NON-BINDING | |
|---|---|---|---|
| 221120 | 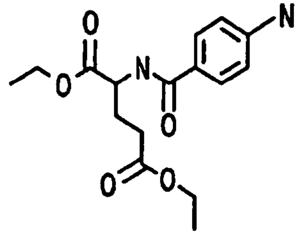 | 108123 | 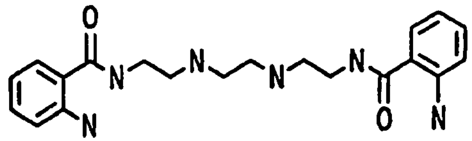 |
| 107146 | 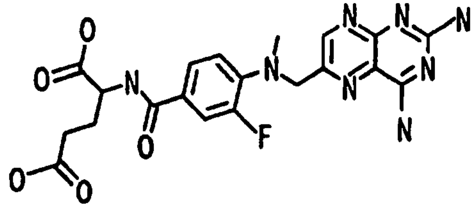 | 339938 | 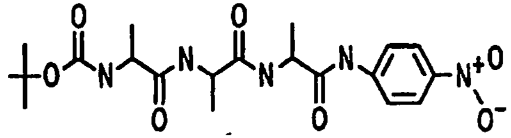 |
| 145882 | 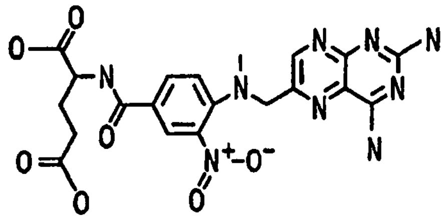 | V8878 | 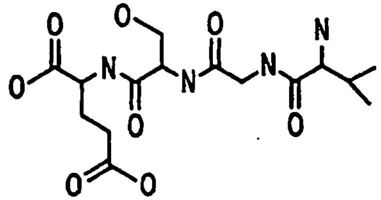 |
| 161613 | 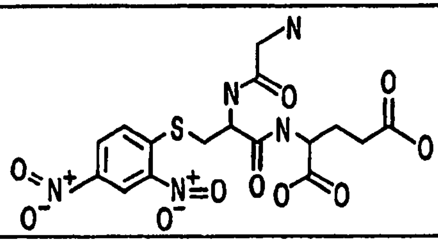 | 579270 | 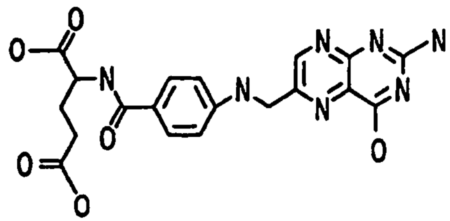 |

*FIG. 8*

CHEMDIV COMPOUNDS

BINDING

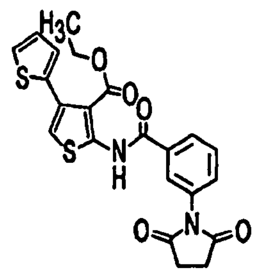

| | | | |
|---|---|---|---|
| 3237-0565 | $C_{22}H_{18}N_2O_5S_2$ | | |
| | 464.5272 | | CSC |

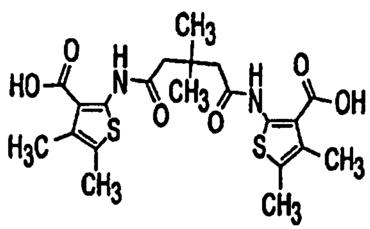

| | | | |
|---|---|---|---|
| 3237-0713 | $C_{21}H_{26}N_2O_6S_2$ | | |
| | 466.5792 | | CSC |

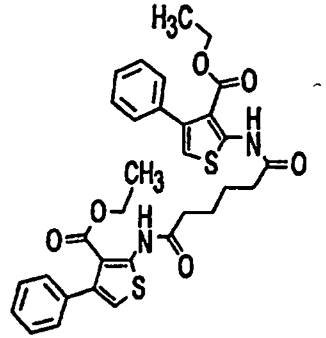

| | | | |
|---|---|---|---|
| 3237-0430 | $C_{32}H_{32}N_2O_6S_2$ | | |
| | 604.7496 | | CSC |

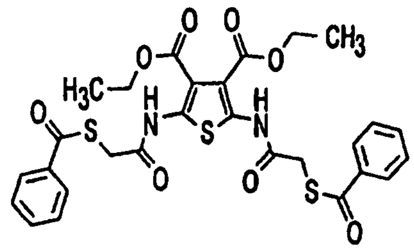

| | | | |
|---|---|---|---|
| 8006-2560 | $C_{28}H_{26}N_2O_8S_3$ | | |
| | 614.7200 | | CSC |

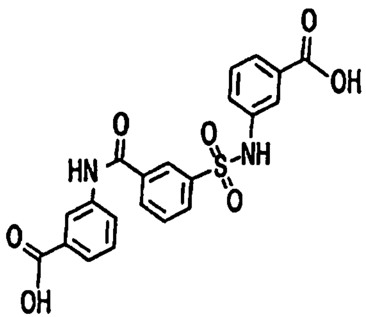

| | | | |
|---|---|---|---|
| 0090-0031 | $C_{21}H_{16}N_2O_7S$ | | |
| | 440.4349 | | CSC |

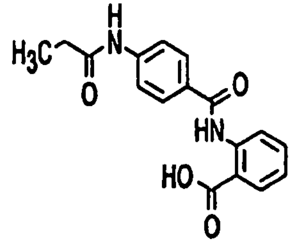

| | | | |
|---|---|---|---|
| 2372-2393 | $C_{17}H_{16}N_2O_4$ | | |
| | 312.3281 | | CSC |

*FIG. 9A*

CHEMDIV COMPOUNDS

NON-BINDING

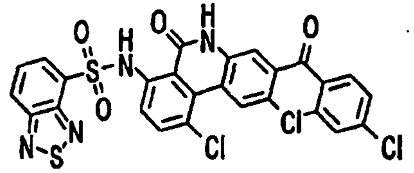

| | | | |
|---|---|---|---|
| 0136-0181 | $C_{26}H_{16}Cl_3N_4O_4S$ | | |
| | 617.8208 | | CSC |

FIG. 9B

CHEMDIV COMPOUNDS

BINDING

8004-1312

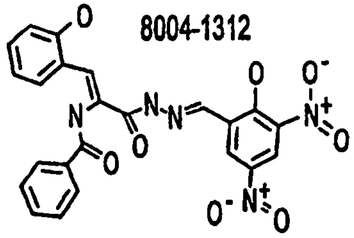

3289-8625

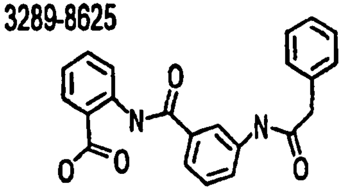

3289-5066

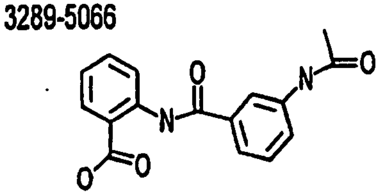

3237-0719

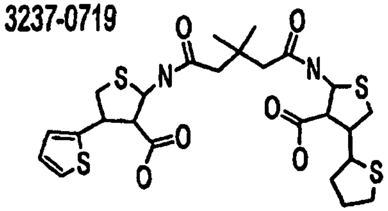

NON-BINDING

8003-2178

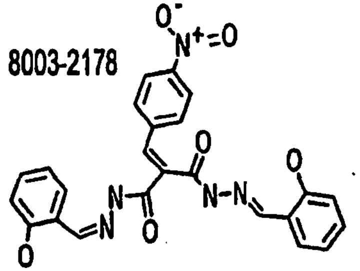

C691-0030

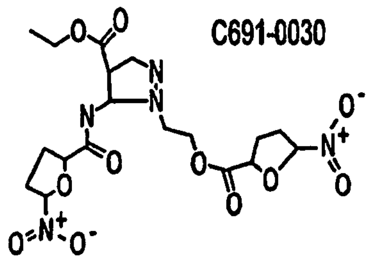

1748-0253

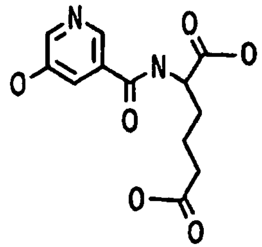

1108-0424

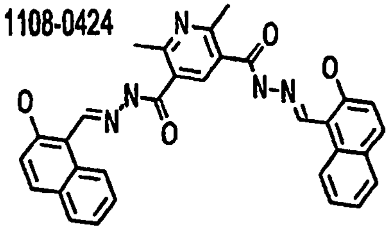

2922-0102

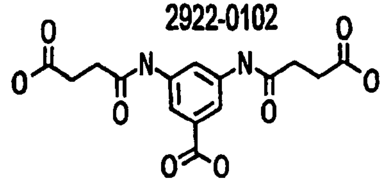

3379-2274

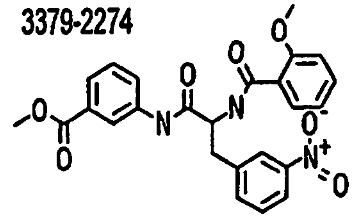

8003-4726

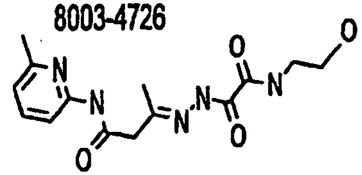

FIG. 10

| | 1 | 2 | 3 | 4 | 5 |
|---|---|---|---|---|---|
| 0 | 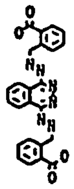 NAME_ 103673 | racemic 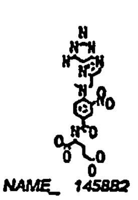 NAME_ 145882 | 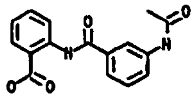 NAME_ 3289_5066 | 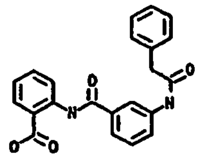 NAME_ 3289_8625 | racemic 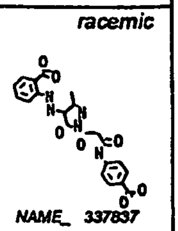 NAME_ 337837 |
| 5 | 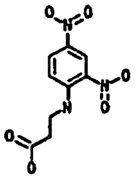 NAME_ 7129 | 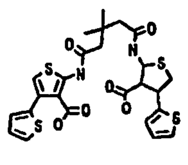 NAME_ 3237_0719 | 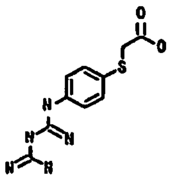 NAME_ 125217 | 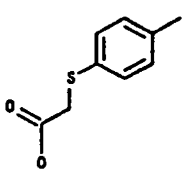 NAME_ P1 | 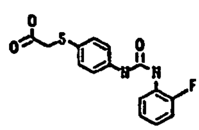 NAME_ 142277 |
| 10 | 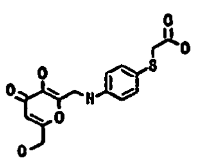 NAME_ 82569 | 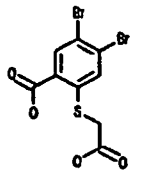 NAME_ 39869 | 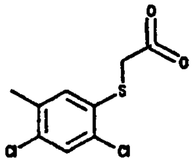 NAME_ P3 | 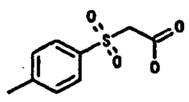 NAME_ 46893 | 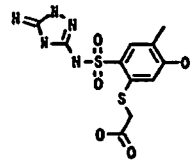 NAME_ 661075 |
| 15 | 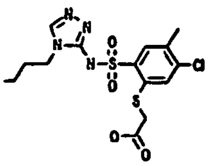 NAME_ 661080 | 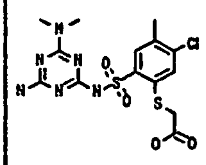 NAME_ 661086 | 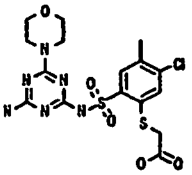 NAME_ 661092 | racemic 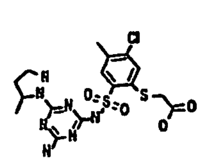 NAME_ 661091 | 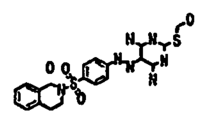 NAME_ 84123 |
| 20 | racemic 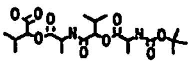 NAME_ 668036 | | | | |

*FIG. 11*

| | | βA | | βB | | βC | | αA | |
|---|---|---|---|---|---|---|---|---|---|
| mDv11 | 251 | TVTLNM | ERHHFLG | ISIV | GQSNDR...GDGG | IYIGSI | MKGG | AVAAD | GRIEPG |
| mDv12 | 267 | TVTLNM | EKYNFLG | ISIV | GQSNER...GDGG | IYIGSI | MKGG | AVAAD | GRIEPG |
| mDv13 | 249 | TVTLNM | EKYNFLG | ISIV | GQSNER...GDGG | IYIGSI | MKGG | AVAAD | GRIEPG |
| dDsh | 252 | TVSINM | EAYNFLG | ISIV | GQSNRG...GDGG | IYVGSI | MKGG | AVALD | GRIEPG |
| xDsh | 254 | TVTLNM | EKYNFLG | ISIV | GQSNER...GDGG | IYIGSI | MKGG | AVAAD | GRIEPG |
| **Class** | I | | * | * | | | | | |
| zo1 | | KLVKFRK | GD..SVG | LRLA | GGND.......VG | IFVAGV | LEDS | PAAKE | G.LEEG |
| zo2 | | KLVKFKK | GD..SVG | LRLA | GGND.......VG | IFVAGI | QEGT | SAEQE | G.LQEG |
| PSD95c | | RRIVIHR | GST.GLG | FNIV | GGEDGE......G | IFISFI | LAGG | PADLS | GELRKG |
| hDLG | | RKIILHG | GST.GLG | FNIV | GGEDGE......G | IFVSFI | LAGG | PADLS | GRLRRG |
| PSD95a | | EEITLER | GNS.GLG | FSIA | GGTDNPHIGDDPS | IFITKI | IPGG | AAAQD | GRLRVN |
| PSD95b | | MEIKLIK | GPR.GLG | FSIA | GGVGNQHIPGDNS | IYVTKI | IEGG | AAHKD | GRLQIG |
| **Class** | II | | * | * | | | | | |
| LIN2 | | RLVQFQK | DTQEPMG | ITLK | VNEDG.......R | CFVARI | MHGG | MIHRQ | ATLHVG |
| hCASK | | RLVQFQK | NTDEPMG | ITLK | MNELN.......H | CIVARI | MHGG | MIHRQ | GTLHVG |
| gpdz7 | | HKVTLYK | DSGMEDFG | FSVA | DGLL......EKG | VYVKNI | RPAG | PGDLG | .GLKPY |

*FIG. 12A*

| | βD | | βE | | αB | | βF | | |
|---|---|---|---|---|---|---|---|---|---|
| mDvl1 | DMLLQV | ND | VN | FENMS | NDDAVRVLRE | IVSQTGPI | SLTVAKA | WDPT | 342 |
| mDvl2 | DMLLQV | ND | MN | FENMS | NDDAVRVLRD | IVHKPGPI | VLTVAKC | WGPS | 358 |
| mDvl3 | DMLLQV | NE | IN | FENMS | NDDAVRVLRE | IVHKPGPI | TLTVAKC | WDPS | 340 |
| dDsh | DMLIQV | ND | VN | FENMT | NEDAVRVLRE | VVQKPGPI | KLVVAKC | WDPN | 343 |
| xDsh | DMLLQV | ND | IN | FENMS | NDDAVRVLRD | IVHKPGPI | VLTVAKC | WDPS | 345 |
| | | | | | * | | | | |
| **Class** I | | | | | | | | | |
| zo1 | DQILRV | NN | VD | FTNII | REEAVLFLLD | LPK...GE | EVTILAQ | KK | |
| zo2 | DQILSV | NT | QD | FRGLV | REDAVLYLLE | LPK...GE | TVTILAQ | SR | |
| PSD95c | DQILSV | NG | VD | LRNAS | HEQAAIALKN | A.....GQ | TVTIIAQ | YK | |
| hDLG | DRILSV | NG | VN | LRNAT | HEQAAAALKR | A.....GQ | SVTIVAQ | YR | |
| PSD95a | DSILFV | NE | VD | VREVT | HSAAVEALKE | A.....GS | IVRLYVM | RR | |
| PSD95b | DKILAV | NS | VG | LEDVM | HEDAVAALKN | T.....YD | VVYLKVA | KP | |
| | | | | | * | | | | |
| **Class** II | | | | | | | | | |
| LIN2 | DEIREI | NG | MS | VANRS | VESLQEMLRD | AR...GQV | TFKIIPS | YR | |
| hCASK | DEIREI | NG | IS | VANQT | VEQLQKMLRE | MR...GSI | TFKIVPS | YR | |
| gpdz7 | DRLLOV | NH | VR | TRDFD | CCLVVPLIAE | S.....GN | KLDLVIS | RN | |

*FIG. 12B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10849643 B
- WO 10849067 A
- WO 2004092346 A